(19) Europäisches Patentamt
European Patent Office
Office européen des brevets

(11) EP 0 539 968 B1

(12) EUROPEAN PATENT SPECIFICATION

(45) Date of publication and mention of the grant of the patent:
**15.07.1998 Bulletin 1998/29**

(51) Int Cl.[6]: **G01N 21/64**

(21) Application number: **92118463.6**

(22) Date of filing: **29.10.1992**

# (54) Exciplex-based sensors and methods for sensing

Exciplexsensoren und Bestimmungsverfahren

Capteurs basés sur un état exciplex et procédés d'analyse

(84) Designated Contracting States:
**AT BE DE DK ES FR GB IT NL SE**

(30) Priority: **31.10.1991 US 786189**

(43) Date of publication of application:
**05.05.1993 Bulletin 1993/18**

(73) Proprietor: **MINNESOTA MINING AND MANUFACTURING COMPANY**
**St. Paul, Minnesota 55133-3427 (US)**

(72) Inventors:
- **Bentsen, James G., c/o Minnesota Mining and St. Paul, Minnesota 55133-3427 (US)**
- **Nagel, Colleen C., c/o Minnesota Mining and St. Paul, Minnesota 55133-3427 (US)**

(74) Representative:
**Hilleringmann, Jochen, Dipl.-Ing. et al**
**Patentanwälte**
**von Kreisler-Selting-Werner,**
**Postfach 10 22 41**
**50462 Köln (DE)**

(56) References cited:
**EP-A- 263 693** **EP-A- 363 219**
**US-A- 5 037 615**

- **PROCEEDINGS S.P.I.E. vol. 990, 1989, pages 116 - 120 A. SHARMA AND O.S. WOLFBEIS 'Fiber-optic fluorosensor for sulfur dioxide based on energy transfer and exciplex quenching'**
- **ANALYTICAL CHEMISTRY vol. 62, no. 22, 15 November 1990, COLUMBUS US pages 2461 - 2466 A. UENO ET AL. 'Host-guest sensory systems for detecting organic compounds'**
- **O.S. WOLFBEIS 'Fiber Optic Chemical Sensors and Biosensors' Volume 2. Chapter 10: 'Oxygen Sensors' 1991 CRC Press, Boca Raton, US pages 19 - 53**
- **SCIENCE vol. 226, no. 4677, 23 November 1984, pages 917 - 921 S.L. MATTES ET AL. 'Exciplexes and electron transfer reactions'**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 539 968 B1

Printed by Jouve, 75001 PARIS (FR)

## Description

The present invention relates to sensing or determining the concentration of an analyte of interest in a medium. More particularly, the invention relates to sensor apparatus or systems and methods for sensing the concentration of an analyte of interest, for example, oxygen, in a medium, for example, blood.

It is sometimes necessary or desirable for a physician to determine the concentration of certain gases, e.g., oxygen and carbon dioxide, in blood. This can be accomplished utilizing an optical sensor which contains an optical indicator responsive to the component or analyte of interest. The optical sensor is exposed to the blood, and excitation light is provided to the sensor so that the optical indicator can provide an optical signal indicative of a characteristic of the analyte of interest. For example, the optical indicator may fluoresce and provide a fluorescent optical signal as described in U.S.-A-RE31,897 or it may function on the principles of light absorbance as described, for example, in U.S.-A-4,041,932.

The use of optical fibers has been suggested as part of such sensor systems. The optical indicator is placed at the end of an elongated optical fiber which is placed in the medium to be analyzed. This approach has many advantages, particularly when it is desired to determine a concentration of analyte in a medium inside a patient's body. The optical fiber/indicator combination can be made sufficiently small in size to easily enter and remain in the cardiovascular system of the patient. Consistent and accurate concentration determinations are obtained.

Luminescence measurement analysis for monitoring concentrations of analytes is well known in the art. Generally, a calibration curve of light intensity (or a function of intensity) vs. concentration of the analyte is made. This method may involve a determination of absolute light intensities of both excitation and emission. When luminescent indicators are excited by an intensity modulated excitation source, the phase shift between the excitation and emission signals can be used to determine an analyte dependent luminescence lifetime.

One problem which may exist in such systems is the wavelength proximity between the excitation signal (light) and the emission signal (light) of the indicator. In many cases, the excitation signal and emission signal each have relatively similar wavelengths. This can result in misinterpreting the emission signal, which misinterpretation results in an inaccurate determination of the analyte concentration. It would be advantageous to provide a sensing system in which the wavelengths of the excitation and emission signals are substantially different.

While substantial differentiation of the excitation and emission signals can be achieved using phosphorescent organic indicators, dye stability is often a problem. Indeed, U.S.-A-3,725,648, explicitly excludes phosphorescent molecules as potential sensor materials because of the stability problems. Some phosphorescent platinum group inorganic complexes and phosphorescent lanthanide complexes have shown suitable stability but are quite expensive and may generate significant amounts of highly reactive singlet oxygen upon irradiation in the presence of oxygen. It would be advantageous to provide a sensing system in which substantial differentiation of excitation and emission signals is achieved using stable dyes with reduced, or no, generation of reactive species, such as singlet oxygen.

Substantial differentiation of the excitation and emission signals can be achieved with an excited singlet state by using energy transfer from an analyte responsive first dye to an analyte insensitive second dye (EP-A-0 381,026). Furthermore, pyrene excimer emission has been successfully employed in solutions for the detection of organic compounds (Ueno et al, Anal. Chem., 1990, 62, 2461-66) and anesthetics (Merlo et al, IEEE Engineering in Medicine & Biology, 11th Annual International Conference Proceedings, 1989), with excimer fluorescence serving as a reporter of the aggregation of cyclodextins or of a change in the local viscosity, respectively. These sensing mechanisms work on the premise of an analyte suppressing or enhancing the efficiency for population of an emissive excited state. These systems are not disclosed as being usable in solid sensing elements. An additional problem with such systems is that they are incompatible with phase modulated detection methods, since the lifetime of the emissive state is substantially independent of analyte concentration.

It would be advantageous to provide a sensor system in which the lifetime, and preferably both the lifetime and intensity, of the differentiated emitted signal are sensitive to analyte concentration in a medium. Emissions, for example, fluorescent emissions, which are sensitive or dependent in terms of both lifetime and intensity to analyte concentration are said to be dynamically quenchable by the analyte.

Fiber-optic based sensors are very useful, for example, in medical applications. One problem which may exist with such systems is related to the inherent flexibility of optical fibers. These flexible fibers have a tendency to bend which, in turn, distorts the signals being transmitted by the fibers to the signal processor. Signal distortion caused by fiber bends or other sensor system problems result in inaccurate concentration determinations. It would be advantageous to provide a sensor and concentration determination method which provide accurate concentration data in spite of such distortions.

U.S.-A-4,548,907 discloses a fluorescence-based optical sensor which includes a fluorophor having an acid form and a base form. The fluorophor is chemically unstable in the presence of the medium. Specifically, the relative amounts of the acid form and base form vary depending on the pH of the medium. The fluorophor is excited at two different wavelengths, one for the acid form and one for the base form, and fluorescence signals at a single wavelength are

detected. By ratioing the fluorescence signals obtained at the two different excitation wavelengths, the pH of the medium can be determined. This sensor has the advantage of using a single fluorphor. However, the sensor of US-A-4,548,907 is limited in that only those analytes which influence the ratio of acid form to base form of the fluorophor can be monitored. Again, the emission lifetimes are substantially independent of analyte concentration. Also, no other multiple state optical indicators are taught or suggested.

Lee, et al in "Luminescence Ratio Indicators for Oxygen", Anal. Chem., 59, p 279-283, 1987, report on work the goal of which was to develop a single reagent that would show two luminescence bands, a shorter wavelength "analytical" band subject to quenching by oxygen and a longer wavelength "reference" band independent of oxygen levels. Specifically, the work was to formulate a system showing both shorter wavelength oxygen-sensitive pyrene monomer emission and longer wavelength oxygen-insensitive pyrene dimer emission. This work did not succeed in finding a ratio-based indicator system to measure oxygen in aqueous systems. Further, as noted above, using a shorter wavelength oxygen sensitive emission can result in oxygen concentration determination inaccuracies because of possible overlapping between this short wavelength emission and the excitation signal.

CA-A-2,015,415 discloses an oxygen sensor including a single species of indicator selected from perylene derivatives dispersed or immobilized in a crosslinked polydimethylsiloxane matrix which gives a shorter wavelength oxygen sensitive emission and a longer wavelength oxygen insensitive emission and, thus, can be used as both the indicator and the reference element. Using shorter wavelength oxygen sensitive emission can result in inaccuracies because of overlap with the excitation signal, as described above. Also, there is no teaching or suggestion that the shorter and longer wavelength emissions are the result of different forms of the indicator. To the contrary, the document implies that a single indicator species provides an oxygen-sensitive emission region and a different oxygen-insensitive emission region.

EP-A-0363219 discloses an oxygen sensing apparatus using Europium or Erythrosin-B as phosphors which are excited with a mono-chromatic light that is sine wave modulated in the kHz regime. The emitted light of a different wavelength is also sine wave modulated, with the phase difference between the two sine waves being a measure of the quenching effect of oxygen and, thus, a measure of the partial pressure of oxygen. This publication does not disclose the use of any other indicators, for example, fluorescent indicators. Modulation in the kHz region cannot be extended to shorter lived fluorescent indicators because the phase offsets introduced by transmission of the excitation and emission signals, e.g., through an optical fiber, become significant. Also, there is no teaching or suggestion that the phosphors used produce different emitting forms. Further, as noted above, the use of phosphorescent indicators can result in other problems.

Sharma et al in "Unusually Efficient Quenching of the Fluorescence of An Energy Transfer-Based Optical Sensor for Oxygen", Analytica Chimica Acta, 212 (1988) 261-265, discloses a two fluorophore system consisting of pyrene as energy donor and perylene as energy acceptor dispersed in silicone rubber. A strong fluorescent signal, which is sensitive to oxygen quenching, is emitted at 474 nm, where pyrene does not fluoresce but where the relatively oxygen insensitive perylene does fluoresce. This emitted signal is believed to result in part from a mixed pyrene/perylene excimer. No covalent bonding of pyrene and perylene to the silicone rubber is disclosed so that there is not teaching or suggestion as to how such covalent bonding affects the system. Also, since perylene fluoresces at the same wavelength as does the mixed excimer, the signal emitted from the perylene may interfere with the signal from the mixed excimer. It would be advantageous to employ an emitted signal for analyte concentration determinations which is substantially removed or resolved from other emissions in the system.

PROCEEDINGS SPIE, vol. 990, pages 116-120 discloses an optical sensor based on a transfer of electromagnetic energy from a donor to an acceptor. Energy transfer is a collisionless process that occurs through space and requires substantial optical overlap between the characteristic emission of the first dye and the characteristic absorption of the second dye. The first dye absorbs light at a characteristic excitation wavelength and its excited state energy is transferred to the second dye. In this way, the second dye can emit at its own characteristic emission wavelength in response to excitation of the first dye. Oxygen, in such systems, is believed to quench the excited state of the first indicator (e. g. pyrene) thereby reducing energy transfer efficiency and the resultant emission from the second indicator (e.g. perylene).

It is the object of the present invention to provide a sensor and a method for sensing the concentration of a component or analyte of interest in a medium, which sensor or method give accurate, reliable, and reproducible concentration determination.

According to the invention, this object is solved by the subject matter of claims 1, 16, and 25, respectively. The subject matter of the subclaims refers to preferred embodiments.

As used herein, the term "monomeric indicator component" refers to a monomeric component which provides a signal, preferably an optically detectable signal, in response to being exposed to an excitation signal, preferably an excitation light signal. A "fluorescent monomeric indicator component" is a monomeric indicator component which provides a fluorescence signal in response to being exposed to an excitation signal.

As used herein, the term "monomeric component" refers to a species which may or may not itself provide a signal,

such as an optically detectable signal, in response to being exposed to an excitation signal, such as an excitation light signal.

As used herein, the term "monomeric non-indicator component" refers to a monomeric component which is incapable of providing an effective signal, such as an optically detectable signal, in response to being exposed to an excitation signal, such as an excitation light signal.

As used herein, the term "exciplex component" refers to an excited state species derived from a molecular or submolecular interaction of two or more, preferably two, different monomeric components at least one of which is a monomeric indicator component; one or more preferably one, of which is a donor component and one or more, preferably one, of which is an acceptor component; and which satisfies the following relationship:

$$E_D^{ox} - E_A^{red} \leq h\upsilon \text{ (hex)} + 0.25\text{eV}$$

where $E_D^{ox}$ is the ground state oxidation potential of the donor component, $E_A^{red}$ is the ground state reduction potential of the acceptor component, each measured relative to the same standard, $h\upsilon$ (hex) is the maximum energy in eV (electron volts) of the excited state species measured in n-hexane, and the donor component and acceptor component are assigned so that the absolute value of $E_D^{ox} - E_A^{red}$ is minimized.

The present invention provides sensors and methods for sensing the concentration, for example, partial pressure, of a component or analyte of interest, such as oxygen, in a medium, for example, an aqueous-based medium, such as blood. The present systems function to give accurate, reliable and reproducible concentration determinations. In addition, such determinations can be provided in spite of signal transmission problems, such as, bent optical fibers, and other operational difficulties which may affect the quality of the signals being transmitted. Further, efficient utilization of the indicator component or components is achieved often resulting in sensors having reduced indicator component loadings. Moreover, these advantageous results are achieved using sensors, equipment and techniques which are relatively simple, easily operated and conveniently executed.

In one broad aspect, the present sensors comprise a sensing element, an excitation assembly and a detector assembly. The sensing element includes two or more different monomeric components at least one of which is a monomeric indicator component, each of which monomeric components is preferably located in, more preferably covalently bonded to, a matrix material, preferably a solid matrix material. The monomeric indicator component is capable of providing a first emitted signal of a given wavelength in response to being exposed to a first excitation signal. This sensing element is capable of providing a second emitted signal, preferably having a longer wavelength than the first emitted signal, in response to being exposed to the second excitation signal. Preferably, the first excitation signal and the second excitation signal are the same signal. The second emitted signal is provided by an exciplex component produced, for example, in the sensing element, from the monomeric components and is preferably dependent on, i.e., varies in response to changes in, analyte in the medium to which the sensing element is exposed. More preferably, this second emitted signal is more dependent on the concentration of the analyte in the medium to which the sensing element is exposed than the first emitted signal. The exciplex component derived second emitted signal preferably is dynamically quenched or subject to being dynamically quenched by the analyte. Exciplex component emissions which are dynamically quenchable are preferred because, for example, they are useful in both intensity-based and phase modulated sensing systems.

The excitation assembly is positioned and adapted to provide one or more excitation signals to the sensing element. Such excitation signals preferably each have a wavelength which is shorter than the first emitted signal. The first and second excitation signals may each have substantially the same wavelength.

The detector assembly is positioned and adapted to detect either the second emitted signal from the sensing element, or the first emitted signal and the second emitted signal from the sensing element.

The processor assembly is preferably positioned and adapted to process or analyze either the second emitted signal in determining the concentration of the analyte in the medium, or the first emitted signal and the second emitted signal in determining the concentration of the analyte in the medium.

Using the exciplex component provided second emitted signal as at least part of the basis for the analyte concentration determination provides substantial benefits. This second emitted signal is different and distinct from the first emitted signal provided by the monomeric indicator component. This second emitted signal preferably has a relatively long wavelength and, thus, is further shifted away (red-shifted) from the excitation signal or signals than is the first emitted signal. Thus, there is reduced risk of misinterpreting the second emitted signal (for example, because of interference from the excitation signal or signals) than of misinterpreting the relatively shorter wavelength first emitted signal. Employing relatively long wavelength exciplex component provided second emitted signals which are more analyte sensitive than the first emitted signal provided by the monomeric indicator component results in increased indicator utilization efficiency. That is, the presence of such analyte sensitive longer wavelength second emitted signal means that the formation of the exciplex component is advantageously kinetically favored so that relatively less monomeric indicator component is needed to form the same amount of exciplex component (relative to a system using shorter wavelength emissions which are more analyte sensitive). Thus, the amount of monomeric components can be reduced and/or even the size of the sensor can be reduced using the preferred longer wavelength analyte sensitive exciplex component provided emitted signals of the present invention. The use of the exciplex component provided second emitted signal results in accurate, reliable, reproducible and efficient analyte concentration determinations.

A particularly useful embodiment involves a sensing element which provides an intensity modulated, preferably a sine wave modulated, second emitted signal in response to being exposed to an intensity modulated, preferably a sine wave modulated, second excitation signal. This modulated second emitted signal is provided by an exciplex component produced in the sensing element. The processor assembly is positioned and adapted to analyze the modulated second emitted signal and the modulated second excitation signal in determining the concentration of the analyte in the medium. The extent of the phase shift and/or the magnitude of relative demodulation between the modulated second excitation signal and the modulated second emitted signal is/are dependent on the concentration of the analyte in the medium. As used herein, the term "relative demodulation" refers to the demodulation factor for the modulated emitted signal with respect to the modulated excitation signal which results in the modulated emitted signal. Specifically, in this instance, relative demodulation is the demodulation factor for the modulated second emitted signal with respect to the modulated second excitation signal. The use of the exciplex component provided modulated second emitted signal as a basis for analyte concentration determinations provides substantial benefits, for example, in terms of very good analyte concentration accuracy determination. The modulated signals referred to herein may be modulated in the MHz range, as opposed to being limited to the kHz range as disclosed in the European Patent Publication noted above. The use of the extent of the phase shift and/or the magnitude of relative demodulation, as described herein, at a variety of modulation frequencies can be used to determine analyte concentration, and is included within the scope of the present invention.

In one embodiment, the processor assembly is adapted to determine the ratio of the second emitted signal to the first emitted signal (or vice versa). Such a signal ratio is itself preferably dependent on the concentration of the analyte in the medium. The first emitted signal or signals may also be dependent on the concentration of the analyte in the medium.

In another embodiment, the first emitted signal and the second emitted signal are intensity modulated, preferably sine wave modulated, and the processor assembly is adapted to determine at least one of the extent of the phase shift between and the ratio of demodulation factors of the modulated first emitted signal and the modulated second emitted signal. The extent of this shift and/or this ratio is/are dependent on the concentration of the analyte in the medium.

Using such a signal ratio or both the modulated first emitted signal and the modulated second emitted signal in determining the analyte concentration reduces, or even substantially eliminates, the detrimental effect on the accuracy of the concentration determination caused by, for example, distortion in the signals, for example, as the result of bent optical fibers. One particular advantage of exciplex component forming systems over internally referenced systems containing a separate second indicator dye is that the analyte sensitive emission and the reference emission can be well resolved from one another using a single excitation wavelength. Furthermore, the intensity ratio, or the phase difference or the demodulation factor ratio methods can provide for linear Stern-Volmer calibration slopes.

The use of modulated signals, as described herein, is intensity independent. That is, the use of an analyte sensitive extent of shift in modulated signals is applicable regardless of the intensity of the signals used. The use of such modulated signals provides for very reliable and reproducible analyte concentration determinations.

For the specific embodiments described herein, the excitation assembly, detector assembly and processor assembly may be chosen from equipment which is conventional and well known in the art.

In yet another broad aspect, the invention involves methods for sensing the concentration, for example, partial pressure, of an analyte, for example, oxygen or other normally gaseous component, in a medium, such as blood or other aqueous, for example, liquid aqueous, medium. These methods comprise: exposing a sensing element, as described herein, to the medium; causing the sensing element to provide a second exciplex component provided emitted signal, or first monomeric indicator component provided emitted signal and a second exciplex component provided emitted signal, as described herein; and analyzing the second emitted signal or the first emitted signal and the second emitted signal in determining the concentration of analyte in the medium. The excitation signal or signals may also be analyzed in these methods.

A still further broad aspect of the present invention is the provision of compositions, for example sensing elements, useful for measuring the concentration of an analyte in a medium. In one embodiment, the compositions comprise effective amounts of two or more monomeric components, which are preferably covalently linked or bonded together, at least one of which is a monomeric indicator component. The compositions may include a matrix material, preferably a solid matrix material, which is permeable to the analyte in the medium. The monomeric components may be in, for example, within and/or on, preferably within, the matrix material. The monomeric indicator component is capable of providing a first emitted signal in response to being exposed to a first excitation signal. In one particularly useful embodiment, the monomeric components are covalently linked or bonded together other than through the matrix material.

The covalent bonding between the monomeric components is preferably by a linkage which facilitates the appropriate exciplex component-forming interaction between these monomeric components. The composition is capable of providing a second emitted signal in response to being exposed to the second excitation signal. This second emitted signal is provided by an exciplex component produced from the monomeric components. The second emitted signal is preferably dependent on the concentration of the analyte in the medium. This second emitted signal is preferably dynamically quenchable by the analyte in the medium.

The first emitted signal is preferably also dependent on the concentration of the analyte in the medium. Preferably, the second emitted signal is dependent on the concentration of the analyte in the medium to a greater extent than is the first emitted signal. The monomeric components are preferably covalently bonded to the solid matrix material. In a particularly useful embodiment, the solid matrix material is a silicone-based polymer. A particular example of such composition involves those systems in which the monomeric components are covalently tethered to each other in such a manner to facilitate the production of the desired exciplex component. In one particularly useful embodiment, the number of atoms, for example, carbon atoms, in the chain covalently linking the monomeric components together is in the range of about 1 to about 20, more preferably about 2 to about 7.

The present exciplex component provided emitted signals are preferably dependent on the concentration of the analyte in the medium, more preferably to a greater extent than the other emitted signal or signals from any given sensor of the present invention. Such exciplex component provided emitted signals are preferably dynamically quenchable by the analyte in the medium. The other emitted signal or signals from any given sensor in accordance with the present invention may also be dynamically quenchable by the analyte in the medium. In one embodiment, the signal or signals emitted from the sensing element preferably result from the sensing element fluorescing. In this embodiment, the Stern-Volmer quenching constant of the exciplex component provided emitted signal is preferably higher than that of the other emitted signal or signals.

The sensors, methods and compositions of the present invention are useful in applications where the analyte is not a dynamic quencher of the emitted signal or signals, but instead, where the analyte concentration can be made to affect the concentration of a messenger species which is capable of dynamically quenching the emitted signal or signals. For example, it is well known in the art that enzymatic reactions involving glucose, adenosine triphosphate (ATP) or cholesterol dependent production or consumption of oxygen enable oxygen optrodes to serve as transducers for detection of these analytes.

Although the monomeric components may be physically mixed or dispersed in a matrix material of the sensing element, it is preferred that such monomeric components be covalently bonded to the matrix material. For example, such monomeric components may be covalently bonded to, and therefore an integral part of, the polymeric material which is preferably included in the matrix material. Although a monomeric indicator component may be part of a polymer molecule which includes more than one monomeric component moiety, such component is still considered to be monomeric. For example, a monomeric indicator component which is part of a polymer molecule which includes more than one monomeric indicator component moiety is still considered monomeric since it is capable of providing an emitted signal which is characteristic of the basic indicator compound (monomer) from which it is derived. In contrast, the emitted signal provided by the exciplex component produced from the monomeric indicator component and monomeric non-indicator component moieties, for example, on the same polymer molecule and/or on different polymer molecules, has a characteristic which is different from the emitted signal provided by the monomeric indicator component.

The exciplex component is preferably produced when an excitation signal is provided to the sensing element. The exciplex component is believed to be inactive and/or to not be produced in the absence of the excitation signal.

In many instances, the covalently bonded monomeric components are each derived from a compound or substance which itself is not suitable to be covalently bonded to the matrix material. In these instances, it may be necessary to derivatize or functionalize such compound and produce a precursor of the monomeric component. This is done by chemically modifying the compound to include at least one group with a functional portion, preferably a functional multiple bond, which functional portion is capable of chemically reacting with the matrix material or precursor of the matrix material to covalently bond the monomeric component thereto. Of course, if the basic compound from which the monomeric component is derived to be covalently bonded to the matrix material or matrix material precursor, it is not necessary to further derivatize or functionalize such compound.

The functionalizing of such non-covalently bondable compounds is illustrated by selecting a matrix material comprising a silicone-based polymer and monomeric compounds which do not react with the matrix material or matrix material precursor. Such compounds are not able to be covalently bonded to such silicone-based polymers or their precursors. However, these compounds can be reacted to form attachment groups having a functional portion, such as appropriately configured alkenyl groups, substituted alkenyl groups and the like, which are capable of reacting with and bonding to silicone-based polymers and/or precursors thereof, such as polymethylhydrosiloxanes. In particular, if the silicone-based polymer is to be derived by addition curing, it is preferred to functionalize or derivatize the monomeric compounds to attach one or more alkenyl groups and/or substituted alkenyl groups thereto. Such groups are capable of being hydrosilylated, for example, with a polymethylhydrosiloxane to covalently bond the monomeric component to

the silicone-based polymer precursor. The resulting precursors or compounds can be reacted with vinyl-terminated polysiloxane, thereby forming an addition-cure silicone including the monomeric components.

Any type of group may be attached to the monomeric compounds, provided that such group is with a functional portion, preferably a multiple bond, more preferably a carbon-carbon multiple bond and still more preferably a carbon-carbon double bond, which is capable of chemically reacting with a polymer or polymer precursor to form the covalently bonded monomeric components. The group also should have substantially no undue detrimental effect on the analyte sensitivity of the exciplex component provided emitted signal, on the other emitted signal or signals used in making analyte concentration determinations, or on the medium to which the sensing element is exposed. Preferably, the group is organic in nature. Particularly attractive benefits are obtained when the monomeric indicator component is derived from a monomeric indicator component precursor including at least one indicator compound or substance which has an aromatic ring to which is directly covalently bonded a group with a functional multiple bond which is isolated by a silicon-free chain (i.e., a chain of atoms linking the aromatic ring to the functional multiple bond which includes no silicon atoms), more preferably a chain linking only carbon atoms, from the aromatic ring. Such indicator component precursors are relatively easy to produce and use, and provide sensing elements with very useful properties. In another particularly useful embodiment, the group is a vinyl group covalently bonded directly to an aromatic ring of the indicator compound.

As noted above, particularly useful groups include alkenyl groups; substituted alkenyl groups and the like. Such groups and substituted groups preferably include 2 to about 20 carbon atoms, and may have a terminal double bond, i.e., a double bond associated with the terminal carbon atom. Examples of useful groups include vinyl, allyl, butenyl, hexenyl, heptenyl, octenyl, decenyl and the like groups. The presently useful substituted alkenyl groups include the groups described herein substituted with one or more substituent groups including elements such as oxygen, nitrogen, carbon, hydrogen, silicon, halogen, phosphorus and the like and mixtures and combinations thereof. Thus, the attached group can include at least one heteroatom.

Various chemical modification techniques, many of which are conventional and well known in the art, may be employed to functionalize or derivatize the monomeric compounds with the group or groups to produce the monomeric component precursors. Care should be exercised to avoid destroying or even substantially diminishing the analyte sensitivity (e.g., to the gas component of interest) of the presently useful emitted signal or signals in the process of attaching one or more groups. However, it has been found that sufficient sensitivity is maintained if the characteristic structure of the monomeric compound or compounds remains substantially unaffected, i.e., intact, after the chemical modification.

In a particularly useful embodiment, the compound from which the monomeric indicator component is derived is sensitive to the concentration of oxygen and is one or more polynuclear aromatic compounds and/or one or more derivatives thereof. The polynuclear aromatic compound is preferably any fluorescent or absorbent, more preferably fluorescent, optical indicator of the polynuclear aromatic class. The polynuclear aromatic compound from which the monomeric indicator component is derived is still more preferably selected from the group consisting of perylene, derivatives of perylene, decacyclene, derivatives of decacyclene, benzoperylene, for example, benzo[ghi]perylene, derivatives of benzoperylene, for example, derivatives of benzo[ghi]perylene, coronene, derivatives of coronene, pyrene, derivatives of pyrene, porphycine, derivatives of porphycine, porphyrin, derivatives of porphyrin, chlorin, derivatives of chlorin, pthalocyanine, derivatives of pthalocyanine and mixtures thereof. Excellent results are achieved if the polynuclear aromatic compound is benzo[ghi]perylene.

If desired, the basic polynuclear aromatic compound may be derivatized with one or more other groups, e.g., non-functional substituent groups such as alkyl groups, provided such derivatization does not substantially interfere with exciplex component provided emitted signal generation. Such derivatives are discussed in U.S.-A-4,849,172 which is hereby incorporated in its entirety by reference herein. One goal of the use of such derivatives is to increase the solubility of the indicator substance in the matrix material. The "covalent bonding" feature described herein mitigates against this solubility constraint. Thus, the basic or underivatized polynuclear aromatic compounds, e.g., as described herein, may be advantageously used to produce the covalently bonded monomeric component or components. When one or more of the covalently bonded monomeric components is derived from a polynuclear aromatic compound (even an underivatized polynuclear aromatic compound) it is herein considered a derivative of a polynuclear aromatic compound because the polynuclear aromatic compound may include at least a portion of the attached group, noted above, and is derivatized by being covalently bonded to the matrix material. Thus, for example, a monomeric component of a sensing element derived by covalently bonding vinyl benzo[ghi]perylene in an addition cure silicone polymer is said to be a derivative of benzo[ghi]perylene.

The monomeric components useful in the present invention may be selected from any compound or derivatives thereof which is capable of functioning as a monomeric component, as described herein. The monomeric components useful in a given sensor may include two or more different monomeric indicator components, or one or more, preferably one, monomeric indicator components and one or more, preferably one, monomeric non-indicator components. Preferably, such monomeric components have no substantial detrimental effect on the sensing element, on the sensor

system, on the analyte or on the medium to which the sensing element is exposed.

Examples of monomeric components which produce useful donor-acceptor (DA) exciplex components include polynuclear aromatic acceptor monomeric components and aliphatic or aromatic amine-containing or aromatic ether-containing donor monomeric components. Examples of useful aromatic acceptor monomeric components included biphenyl, naphthalene, phenanthrene, p-terphenyl, chrysene, benzpyrene, pyrene, dibenzanthrene, benzanthrene, anthracene, perylene, benzperylene, fluoranthene, coronene, quinoline, phenylquinoline, benzquinoline, quinoxaline, dibenzquinoxaline, benzquinoxaline, phthalimide, pyridine, phenazine, dibenzphenzine, acridine, benzacridine and derivatives of these compounds. Examples of useful donor monomeric components include tetramethyl-p-phenylenediamine, dimethoxydimethylaniline,methoxydimethylaniline,diethylaniline,diphenylmethylamine, triethylamine, indole, dimethyltoluidine, tri-p-anisylamine, ditolymethylamine, tritolylamine, triphenylamine, ethylcarbazole, trimethoxybenzene, tetramethoxybenzene and derivatives of these compounds.

Examples of monomeric components which produce useful acceptor-donor (AD) exciplex components include aromatic nitrile acceptor monomeric components and donors taken from the list of polynuclear aromatic acceptor monomeric components or from the list of DA donors listed in the preceding paragraph. Examples of aromatic nitrile acceptor monomeric components include benzonitrile, cyanonaphthalene, dicyanobenzene and derivatives of these compounds.

Any of these DA or AD pairs can be tethered and/or covalently bonded to a matrix material. Alternatively, freely diffusing DA or AD pairs or their tethered derivatives can be used for sensing applications in free solution.

Preferably, the exciplex component producing monomeric components of the present sensing elements are positioned or oriented so as to facilitate exciplex component formation. Thus, these monomeric components can be said to be in enforced association. By "enforced association" it is meant that the monomeric components are positioned or oriented in physical and/or molecular enforcement to promote or facilitate the formation and/or maintenance of a channel or path for exciplex component formation which (a) is kinetically dominant relative to free diffusion, and/or (b) is kinetically competitive with the decay of the exciplex component in the absence of the analyte. Enforced association facilitates the development of sensors based on a wide variety of monomeric components which may not otherwise form appreciable exciplex at similar concentrations as free monomeric components. Enforced association provides relatively long lived exciplex components with acceptable Stern Volmer slopes in cases where the monomeric indicator components are too short lived to be useful. Enforced association may minimize kinetic heterogeneity for exciplex component disassociation, thereby affording more reproducible Stern Volmer slopes based on the exciplex component to monomeric indicator component ratio, or the phase shift difference, or the demodulation factor ratio, or the exciplex component emission alone.

Enforced association can be achieved in various ways, for example, by:

1. Intramolecular exciplex component forming molecules dispersed in a matrix material.
2. Intramolecular exciplex component forming molecules covalently attached to a matrix material.
3. Monomeric component aggregates dispersed in or covalently attached to a matrix material or adsorbed, preferably at high loadings, to the surface of a matrix material.
4. Intramolecular exciplex component forming molecules covalently attached, adsorbed or otherwise attached to the surface of a matrix material.
5. An inclusion complex comprising the acceptor (donor) in a supra-molecule having an associated donor (acceptor).
6. Ionic binding of ionically charged monomeric components.

A particularly useful embodiment involves two or more different monomeric components at least one of which is a monomeric indicator component, which components are preferably covalently bonded together. The monomeric indicator component is capable of emitting a signal in response to being excited. These monomeric components are capable of producing an exciplex component. By covalently bonding or tethering the two components together, the monomeric components are positioned to have increased accessibility to each other for exciplex component formation. The covalent link between the monomeric components is preferably such that the formation of an exciplex component from the covalently linked components is facilitated. Such covalent link preferably includes about 1 to about 20, more preferably about 2 to about 7, atoms, for example, carbon atoms, in the chain between the components.

The monomeric components, as described above, are preferably covalently bonded to a solid matrix material.

The covalent link between the monomeric components can include a functional portion to enable the monomeric components to be covalently linked through a single covalent link to the solid matrix material. The use of combined monomeric components such as the covalently bonded or tethered combined monomeric components described above, provides for reproducible and reliable analyte concentration determinations by generating reproducible working curves which are independent of combined monomeric component concentration over a broad concentration range.

The amount of monomeric components employed in the present systems may vary over a broad range and depends, for example, on the particular components being employed, on the matrix material employed, on the sensing application involved and the like. Such amount should be effective to produce the desired exciplex component and to yield the desired signal or signals. For example, the total amount of monomeric components may be in the range of about 0.0001% or about 0.001% to about 10% or about 20% or more, by weight calculated on the total weight of the sensing element. In many instances, concentrations of less than about 5% or even about 1% or less, by weight calculated on the total weight of the sensing element, provide excellent results. Care should be exercised, for example, if it is desired (as it is preferred) to achieve enforced association, to avoid conditions which result in extended aggregates or extended aggregation of such components which can cause additional quenching of the exciplex component emission (independent of the analyte concentration) and/or a reduction in analyte sensitivity of the exciplex component emission.

Any suitable matrix material, preferably a polymeric matrix material, may be employed provided that it functions as described herein. Particularly useful polymeric matrix materials include those based on addition cure silicone polymers. The matrix material, or the precursor thereof, should preferably be such as to chemically react with the precursors of the monomeric components and produce a sensing element with covalently bonded monomeric components.

Although various polymers can be employed as the matrix material, it is preferred that the matrix material be permeable, more preferably highly permeable, to the analyte, for example, a normally gaseous component, of interest so that the sensitivity of the sensing element to the analyte of interest is optimized. If a silicone-based polymer is employed in the matrix material, it may include polymers derived from vinyl terminated polysiloxanes and polyalkyl (aryl)hydrosiloxanes. Such polyalkyl(aryl)hydrosiloxanes include, but are not limited to, those having the formula

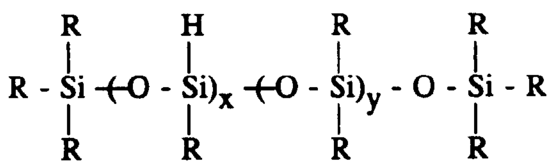

where each of x and y is independently an integer in the range of 1 to about 500 and R is independently selected from the group consisting of H, alkyl, and substituted alkyl, preferably $CH_3$, $CH_2\ CH_2\ CF_3$, $CH_2(CH_2)_nCH_3$, and phenyl, where n is an integer in the range of 1 to about 22. Of this group, polymers in which a major portion of the R groups are methyl are preferred because of the high gas permeability of such polymers. A sufficient number of hydride groups should be present to provide a satisfactory cross-linked or cured polymer, and preferably to react with the monomeric component precursors to covalently bond the monomeric components to the matrix material. It is of course realized that other members of the homologous series which include the above-noted polymers may also be used. The final silicone-based matrix material is cross-linked. Suitable vinyl terminated polysiloxanes include two or more functional vinyl groups which react with the hydride or hydro groups of the polyalkyl(aryl)hydrosiloxanes, for example, polymethylhydrosiloxanes, to form the cross-linked matrix material. Such cross-linking advantageously occurs in the presence of a catalyst, such as a platinum-containing catalyst. The properties of the cross-linked silicone can be varied by changing the degree of cross-linking, for example, by adjusting the concentration of the Si-H groups or component on the polyalkyl(aryl)hydrosiloxanes, for example, polymethyl-hydrosiloxanes, and/or the molecular weight of the vinyl-terminated polysiloxanes.

The precursors of the monomeric components useful in the present invention can be obtained using synthesizing procedures, such as formation of aldehydes, Wittig reactions to give vinyl derivatives, and the like. The monomeric component precursors thus obtained can be dispersed in a silicone-based polymer, such as polymethylhydrosiloxane, in a volatile solvent, such as benzene, hexane and the like, and be allowed to react to covalently bond the monomeric components to the silicone-based polymer. The silicone-based polymer, having the chemically attached monomeric components, is then reacted, for example, using conventional addition curing, to form the sensing element.

An alternative, and less desirable, method for producing the present sensing element involves combining the precursors of the monomeric components with the vinyl-terminated polysiloxane, preferably in an inert solvent to promote dissolution of the above-noted precursors, and any catalyst e.g., platinum group metal, which may be employed. This combination is then mixed with polymethylhydrosiloxane at conditions effective to covalently bond the monomeric components to the matrix material and form the present sensing element.

These and other aspects and advantages of the present invention are set forth in the following detailed description and claims, particularly when considered in conjunction with the accompanying drawings.

Fig. 1 is a schematic illustration of one embodiment of the sensor apparatus according to the present invention.

Fig. 2 is a schematic illustration of an alternate embodiment of the sensor apparatus according to the present invention.

Fig. 1 shows a sensor 10 according to the present invention. Sensor 10 is adapted to determine the concentration or partial pressure of oxygen in blood. An optical fiber 12 is connected to an appropriate light transmitting apparatus 14, which is capable of transmitting light at 285 nanometers. The light transmitting apparatus 14 generates the excitation light at this wavelength. The optical fiber 12 is also connected to a light receiving apparatus 16, which, in turn, is connected to a conventional electronic processor 17.

Located on the optical surface 18 of the optical fiber 12 is a matrix 20 which is an oxygen permeable material, such as a cross-linked addition cured siloxane polymer. Dispersed in matrix 20 is about 0.01% by weight of 1-(1-naphthyl)-2-(diethylamino) ethane.

This siloxane polymer is obtained by mixing a polymethylhydrosiloxane, such as those described above in which x is equal to about 10 and y is equal to about 19, with a vinyl terminated polysiloxane and a hydrocarbon solution of 1-(1-naphthyl)-2-(diethylamino) ethane in the presence of a platinum catalyst to form a cross-linked siloxane polymer including dispersed 1-(1-naphthyl)-2-(diethylamino) ethane moieties. The vinyl terminated polysiloxane has the following formula

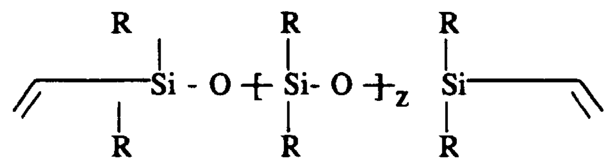

where z is about 78 and each R is methyl. The ratio of SiH to vinyl in the cross-linking reaction is controlled to provide a suitable cross-linked elastomer product. The crosslinking reaction is followed by vacuum drying to remove residual hydrocarbon solvent.

The highly oxygen permeable matrix 20 adheres to the optical surface 18 and slightly down along the sides 22 of the end of fiber 12. An opaque overcoating 24, comprising iron oxide pigment dispersed in an addition cured polysiloxane, can then be applied over the totality of the matrix 20 and down further along the side 22 of the fiber 12.

In use, sensor 10 functions as follows. The tip of optical fiber 12 including matrix 20 and overcoating 24 is exposed or immersed in blood, the oxygen concentration of which is to be determined. Light transmitting apparatus 14 transmits light at 285 nanometers to the optical fiber 12. The excitation light at 285 nanometers causes the matrix 20 to fluoresce at two separate wavelengths, 308 nanometers and 420 nanometers. The emission at the first or shorter wavelength is the result of the excitation of monomeric naphthalene moieties in matrix 20. The emission at the second or longer wavelength is the result of an exciplex which is formed by the interaction of an excited monomeric naphthalene moiety in matrix 20 and one or more unexcited (or ground state) triethylamine moieties in matrix 20. Both the emissions at 308 nanometers and 420 nanometers are dependent on the concentration of oxygen in the blood with the longer wavelength emission being more so dependent than the emission at 308 nanometers.

The fluorescent emitted signals are transmitted from matrix 20 through optical fiber 12 to light receiving apparatus 16. Processor 17 uses information received by light receiving apparatus 16 on the longer emitted signal to determine a value of the oxygen concentration in the blood. Receipt and analysis of this fluorescent light by light receiving apparatus 16 and processor 17 is carried out in a manner similar to that described in the above-referenced Lubbers, et al patent and in Heitzmann U.S. Patent 4,557,900 each of which is incorporated in its entirety herein by reference.

Processor 17 uses information received by light receiving apparatus 16 of the fluorescent signal emitted at 308 nanometers to develop a ratio of the emitted fluorescent signal at 420 nanometers to the fluorescent signal at 308 nanometers. Using this ratio together with the above-noted oxygen concentration, processor 17 can determine a corrected concentration of oxygen in the blood to be analyzed. This corrected oxygen concentration is found to be accurate even if the optical fiber 12 is bent at one or more points along its length and/or if other light transmission difficulties are encountered.

The above-noted procedure may occur periodically or even substantially continuously to give substantially continuous oxygen concentration results. Of course, the transmission of the emission at 308 nanometers can take place before transmission of the emission at 420 nanometers. Also, by proper selection of the optical indicators, e.g., fluorescent dyes, the concentration of other components of interest can be determined. In addition, media other than blood can be analyzed.

The optical fiber 12 may be in the form of a probe or a catheter insertable into a blood vessel of a patient to provide continuous on-line in vivo monitoring of oxygen concentration in the blood. Alternately, the present sensor can be embodied in a flow-through housing as shown, for example, in the above-referenced Heitzmann patent, to provide extra corporeal monitoring of oxygen concentration in the blood.

An alternate embodiment, which is described with reference to Fig. 2, involves a sensor apparatus making use of

intensity modulated (sine wave) signals in the MHz range.

In this embodiment, sensor 110 is adapted to determine the concentration or partial pressure of oxygen in blood. An optical fiber 112 is connected to an appropriate light transmitting apparatus 114, which is capable of transmitting intensity modulated (sine wave) light in the MHz range. The light transmitting apparatus 114 generates the modulated excitation light at this frequency. The optical fiber 112 is also connected to a light receiving apparatus 116, which, in turn, is connected to a conventional electronic processor 117.

The light transmitting apparatus 114 includes a frequency generator (one or more frequencies simultaneously) linked to an electrically controlled light emitting structure, such as a light emitting diode, a frequency doubled light emitting diode, or a combination of elements such as a continuous wave laser or incandescent light source coupled to an acoustooptic modulator or electrooptic modulator, and the like.

The light receiving apparatus 116 includes a highly sensitive light detector having a rapid response time. Suitable detectors include photomultiplier tubes such as those sold under the trademark R928 by Hamamatsu Photonics K.K., Hamamatsu, Japan, as well as avalanche photodiodes and microchannel plates, also available from the same supplier. Using techniques well known in the art, heterodyne detection can be implemented by modulating the detector sensitivity at a frequency, equal to the fundamental modulation frequency, $F_f$ in the MHz regime, plus or minus a heterodyne modulation frequency $F_h$ in the Hz or kHz region.

The processor 117 may include, for example, an analog to digital converter coupled by a direct memory access device to a computer, or an analog phase comparator circuit known to those skilled in the art, and the like. The SLM 48000MHF Fourier Transform Spectrofluometer manufactured by SLM-Aminco in conjunction with an argon ion laser provides frequency modulated light generation, light receiving apparatus and processor capability to perform the methods outlined herein; to measure phase shifts, demodulation factors, or both at either a single modulation frequency or simultaneously at several modulation frequencies. Commercial software is available to apply a well-known digital fast Fourier transform to the data and to interpret phase and demodulation data at multiple modulation frequencies in terms of a distribution of emission lifetimes and relative contributions. This enables determination of the contribution of the exciplex component and monomeric indicator component provided emitted signals to the total phase shift and/or demodulation factors at each wavelength, even when several overlapping emission signals are present.

Located on the optical surface 118 of the optical fiber 112 is a matrix 120 having a composition similar to matrix 20.

The highly oxygen permeable matrix 120 adheres to the optical surface 118 and slightly down along the sides 122 of the end of fiber 112. An opaque overcoating 124, comprising iron oxide pigment dispersed in an addition cured polysiloxane, can then be applied over the totality of the matrix 120 and down further along the side 122 of the fiber 112.

In use, sensor 110 functions as follows. The tip of optical fiber 112 including matrix 120 and overcoating 124 is exposed or immersed in blood, the oxygen concentration of which is to be determined. Light transmitting apparatus 114 transmits light at 10 MHz to the optical fiber 112. This excitation light causes the matrix 120 to fluoresce at two separate wavelengths. Both fluorescent emissions are sine wave modulated. Again the emission at the first or shorter wavelength is believed to be the result of the emission from monomeric naphthalene moieties in matrix 120. The emission at the second or longer wavelength is the result of an exciplex component which is formed by the interaction of one or more excited monomeric naphthalene moieties in matrix 120 and one or more unexcited (or ground state) triethylamine moieties in matrix 120.

The fluorescent emitted signals are transmitted from matrix 120 through optical fiber 112 to light receiving apparatus 116. Processor 117 uses information received by light receiving apparatus 116 on the emitted signals to determine the extent of the phase shift between and/or the ratio of demodulation factors of these two emitted signals. The extent of this phase shift and/or this ratio of demodulation factors are dependent on the concentration of oxygen in the blood. Thus, by determining the extent of this phase shift and/or this ratio of demodulation factors, values of the oxygen concentration in the blood can be obtained.

Alternately, or as a check on the oxygen concentrations obtained by analyzing the two emission signals, processor 117 can use information received from the light transmitting apparatus 114 on the excitation light and information received by light receiving apparatus 116 on the exciplex-derived emitted signal to determine the extent of the phase shift and/or the magnitude of the relative demodulation between this emitted signal and the excitation signal. The extent of this phase shift and/or this magnitude of relative demodulation are dependent on the concentration of oxygen in the blood. Thus, by determining this extent of phase shift and/or this magnitude of relative demodulation, values for the oxygen concentration in the blood can be obtained.

Transmission, receipt and analysis of these modulated signals by light transmitting apparatus 114, light receiving apparatus 116 and processor 117 may be carried out using equipment and in a manner similar to that described in Gratton U.S. Patent 4,840,485 which is incorporated in its entirety herein by reference.

The above-noted procedure may occur periodically or even substantially continuously to give substantially continuous oxygen concentration results. Of course, by proper selection of the optical indicators, e.g., fluorescent dyes, the concentration of other components of interest can be determined. In addition, media other than blood can be analyzed.

The optical fiber 112 may be in the form of a probe or a catheter insertable into a blood vessel of a patient to

provide continuous on-line in vivo monitoring of oxygen concentration in the blood. Alternately, the present sensor can be embodied in a flow-through housing as shown, for example, in the above-referenced Heitzmann patent, to provide extra corporeal monitoring of oxygen concentration in the blood.

The following non-limiting Examples illustrate certain aspects of the invention.

Fluorescence data were obtained on a SPEX Fluorolog Fluorometer, equipped with a 450W Xe lamp, single excitation monochrometer blazed at 250 nm, double emission monochrometer blazed at 500nm. Typical slit widths were 0.5mm or less corresponding to bandwidths on the order of 1 to 2 nm. Emission spectra were corrected and zeroed, excitation spectra were obtained in the ratio mode and were corrected. For variable (oxygen concentration) studies, films were placed in a flow-through chamber equipped with glass or quartz windows. The chamber volume was small to allow for fast gas exchange. The spectra were taken in a front-face mode with excitation normal to the film and emission collected at 22° to the film normal.

Phase modulation data for related experiments were performed using a modified version of the commercially available SLM 48000 Frequency-Domain Fluorometer with single frequency acquisition or with Multi-Harmonic Fourier (MHF) Parallel Acquisition. The SLM 48000 sample chamber was modified by replacing the cuvette holder mount with an x, y, z translator. The terminus of the fiber optic was positioned with the translator of the focal point of the excitation beam (which is focussed with a f/2 lens) such that it was perpendicular to the line of propagation of the excitation. Typically, 200, 600 or 1000 microns core diameter quartz multi-mode fiber optic cables (General Fiber Optics) were used to carry excitation to the sample chamber, and a second fiber of the same core diameter was used to carry emission to the detector. The fibers were cladded with black, electrical cladding to minimize false light effects. The fiber termini were polished and adapted with a Amphenol-type connector on the spectrometer end and a stainless steel capillary tube on the sample end.

EXAMPLE 1

This is an example of the preparation of a film containing an intramolecular exciplex component-forming moiety. To 0.50 g of a vinyl terminated siloxane (sold by Petrarch Systems under the trademark PS441) were added 0.05 g of a polymethylhydrosiloxane (sold by Petrarch Systems under the trademark PS123), 0.50 ml of a $8X10^{-4}$ M $CH_2Cl_2$ solution of 1-(1-naphthyl)-2-(diethylamino)ethane, and 10 microliters of Pt catalyst solution.

The Pt catalyst solution used in these preparations was a solution of Karsted's catalyst in hexane. The solvents used were spectral grade, dried over molecular sieves. 1-(naphthyl)-2-(diethylamino) ethane was prepared according to methods outlined by Chandross and Thomas in Chem. Phys. Letters 9 393(1971). 1-napthylenyl acetic acid was converted to the acid chloride, reacted with diethylamine to make the corresponding amide and reduced to the corresponding amine using lithium aluminum hydride. Extraction from basic aqueous solution into ether gave the desired product in high yield.

The mixture was agitated and poured into a 57 mm diameter aluminum weighing pan and allowed to dry and cure in air, then further dried under vacuum overnight to remove residual solvent. The films thus obtained are optically clear.

The response of this film to oxygen was determined in the intensity mode. Upon being excited at 285 nm, exciplex emission was monitored at 420nm and monomer emission at 308nm. Both exciplex emission and monomer emission were dynamically quenchable by oxygen. When plotted in Stern-Volmer form there was obvious curvature of the exciplex oxygen dependence. However, the exciplex/monomer ratio in a Stern-Vomer treatment produced a linear response with a slope of 0.0025 $mm^{-1}$.

EXAMPLE 2

The response of the film produced in Example 1 is determined in phase. The film is placed in a thermostated optical isolation chamber equipped with a port for collinear excitation and emission optical fibers and ports for rapid gas exchange. The fiber optic port is positioned to provide for excitation and emission collection at an angle of 45 degrees from the film surface to minimize scatter. Emission wavelengths for exciplex emission and for monomer emission are serially selected using a standard monochrometer. The sensor film is exposed to oxygen in nitrogen mixtures with compositions ranging from 0 to 20% oxygen.

Uncorrected phase shifts is obtained from MHF or from single frequency data. These phase shifts can be referenced to the excitation source phase and included phase offsets associated with the electronics and optics.

By plotting $\tan\Delta\theta_o$ /$\tan\Delta\theta$vs $0_2$ concentration, a substantially linear Stern-Volmer slope is obtained. Thus, this exciplex-forming system is useful as a sensing element in an oxygen sensor.

EXAMPLE 3

This is an example involving the use of freely diffusing donor and acceptor molecules in solution to determine the

concentration of an analyte, in this case oxygen.

A solution of 7.2 x $10^{-6}$ M anthracene and 15.7 mM diethylaniline in toluene as solvent was exposed to oxygen in nitrogen mixtures containing 0, 5, 10, 15, 20% by volume oxygen. Anthracene was photoexcited at 379 nm. Monomer emission was monitored at 402 nm. Exciplex emission was monitored at 520 mn. The exciplex/monomer intensity ratio was determined at each oxygen concentration (E/M) and under nitrogen alone $(E/M)_o$. In Stern-Volmer treatment, plots of (E/M)o/(E/M)vs oxygen concentration were linear with a slope of 0.039$mm^{-1}$. Furthermore, this slope was invariant to variations in the concentration of diethylaniline over the range of 15.7-55.0 mM, despite the fact that the exciplex/ monomer ratio itself varied substantially.

This example illustrates that, while the ratio of exciplex to monomer emission intensity varies with donor concentration, conditions can be found where Stern-Volmer slopes based on the exciplex/monomer ratio are independent of donor concentration.Similar behavior can be expected for Stern-Volmer slopes based on the phase difference or demodulation factor ratio methods described in Example 2.

While this invention has been described with respect to various specific examples and embodiments, it is to be understood that the invention is not limited thereto and that it can be variously practiced within the scope of the following claims.

## Claims

**1.** A sensor for measuring the concentration of an analyte in a medium comprising:

- a sensing element (20;120) including a matrix material which is permeable to said analyte in said medium, and two or more different monomeric components in enforced association at least one of which is a monomeric indicator component capable of providing a first emitted signal in response to being exposed to a first excitation signal,
- wherein said two or more different monomeric components are positioned or oriented in physical and/or molecular enforcement to promote or facilitate the formation and/or maintenance of a channel or path for exciplex component formation which (a) is kinetically dominant relative to free diffusion, and/or (b) is kinetically competitive with the decay of the exciplex component in the absence of the analyte,
- said sensing element being capable of providing a second emitted signal in response to being exposed to a second excitation signal, said second emitted signal being provided by an exciplex component produced from said monomeric components,
- wherein an exciplex component refers to an excited state species derived from a molecular or sub-molecular interaction of two or more different monomeric components at least one of which is a monomeric indicator component, one or more of which is a donor component and one or more of which is an acceptor component; and which satisfies the following relationship:

$$E_D^{ox} - E_A^{red} \leq h\nu(hex) + 0.25eV$$

where $E_D^{ox}$ is the ground state oxidation potential of the donor component, $E_A^{red}$ is the ground state reduction potential of the acceptor component, each measured relative to the same standard, hν (hex) is the maximum energy in eV (electron volts) of the excited state species measured in n-hexane, and the donor component and acceptor component are assigned so that the absolute value of $E_D^{ox} - E_A^{red}$ is minimized,
- an excitation assembly (14;114) positioned and adapted to provide said second excitation signal to said sensing element;
- a detector assembly (16;116) positioned and adapted to detect said second emitted signal; and
- a processor assembly (17;117) positioned and adapted to analyze said second emitted signal for determining the concentration of said analyte in said medium.

**2.** The sensor according to claim 1 characterized in that said second emitted signal is dependent on the concentration of said analyte in said medium.

**3.** The sensor according to claim 1 or 2 characterized in that said second emitted signal is dynamically quenchable by said analyte in said medium.

**4.** The sensor according to any one of claims 1 to 3 characterized in that said second emitted signal is dependent on the concentration of said analyte in said medium to a greater extent than is said first emitted signal and said

second emitted signal has a longer wavelength than said first emitted signal.

5. The sensor according to any one of claims 1 to 4 characterized in that said matrix material is solid and said monomeric components are covalently bonded to said solid matrix material.

6. The sensor according to any one of claims 1 to 5 characterized in that said first excitation signal and said second excitation signal are the same signal.

7. The sensor according to any one of claims 1 to 5 characterized in that said second excitation signal and said second emitted signal are modulated, and said processor assembly (17;117) is adapted to determine at least one of

- the extent of the phase shift and
- the magnitude of the relative demodulation between said modulated second excitation signal and said modulated second emitted signal, at least one of said extent and said magnitude being dependent on the concentration of said analyte in said medium.

8. The sensor according to any one of claims 1 to 7 characterized in that said excitation assembly (14; 114) is positioned and adapted to provide said first excitation signal to said sensing element (20;120); said detector assembly (16;116) is positioned and adapted to detect said first emitted signal; and said processor assembly (17;117) is positioned to analyze said first emitted signal and said second emitted signal in determining the concentration of said analyte in said medium.

9. The sensor according to any one of claims 1 to 8 characterized in that said processor assembly (17; 117) is adapted to determine the ratio of said second emitted signal to said first emitted signal, said ratio being dependent on the concentration of said analyte in said medium.

10. The sensor according to any one of claims 1 to 8 characterized in that said first emitted signal and said second emitted signal are modulated and said processor assembly (17;117) is adapted to determine at least one of

- the extent of the phase shift between or
- the ratio of demodulation factors of said modulated first emitted signal and said modulated second emitted signal, at least one of said extent and said ratio being dependent on the concentration of said analyte in said medium.

11. The sensor according to any one of claims 1 to 10 characterized in that said analyte is oxygen.

12. The sensor according to any one of claims 1 to 11 characterized in that said monomeric indicator components in enforced association are selected from the group consisting of intramolecular exciplex component forming molecules dispersed in a matrix material, intramolecular exciplex component forming molecules covalently attached, adsorbed or otherwise attached to the surface of a matrix material, intramolecular exciplex component forming molecules covalently attached to a matrix material, monomeric component aggregates covalently attached to a matrix material, monomeric component aggregates adsorbed to the surface of a matrix material, an inclusion complex comprising a supra-molecule, and ionically bound monomeric components.

13. The sensor according to an one of claims 1 to 12 characterized in said monomeric components are linked by less than 20 atoms.

14. The sensor according to any one of claims 1 to 13 characterized in that said monomeric components are linked by between 2 and 7 carbon atoms.

15. The sensor according to any one of claims 1 to 14 characterized in that the second emitted signal is different and distinct from the first emitted signal.

16. A method for sensing the concentration of an analyte in a medium comprising:

- exposing a sensing element (20;120) to said medium, said sensing element (20;120) including a matrix material which is permeable to said analyte in said medium, and two or more different monomeric components in enforced association at least one of which is a monomeric indicator component capable of providing a first

emitted signal in response to being exposed to a first excitation signal,

- wherein said two or more different monomeric components are positioned or oriented in physical and/or molecular enforcement to promote or facilitate the formation and/or maintenance of a channel or path for exciplex component formation which (a) is kinetically dominant relative to free diffusion, and/or (b) is kinetically competitive with the decay of the exciplex component in the absence of the analyte,
- said sensing element (20;120) being capable of providing a second emitted signal in response to being exposed to a second excitation signal, said second emitted signal being provided by an exciplex component produced from said monomeric components,
- wherein an exciplex component refers to an excited state species derived from a molecular or sub-molecular interaction of two or more different monomeric components at least one of which is a monomeric indicator component, one or more of which is a donor component and one or more of which is an acceptor component; and which satisfies the following relationship:

$$E_D^{ox} - E_A^{red} \leq h\nu(hex) + 0.25eV$$

where $E_D^{ox}$ is the ground state oxidation potential of the donor component, $E_A^{red}$ is the ground state reduction potential of the acceptor component, each measured relative to the same standard, hν (hex) is the maximum energy in eV (electron volts) of the excited state species measured in n-hexane, and the donor component and acceptor component are assigned so that the absolute value of $E_D^{ox} - E_A^{red}$ is minimized,

- causing said sensing element (20;120) to provide said emitted second signal; and
- analyzing said second emitted signal for determining the concentration of said analyte in said medium.

**17.** The method according to claim 16 characterized in that said second emitted signal is dependent on the concentration of said analyte in said medium to a greater extent than is said first emitted signal and said second emitted signal has a longer wavelength than said first emitted signal.

**18.** The method according to claim 16 or 17 characterized in that said matrix material is solid and said monomeric components are covalently bonded to said solid matrix material.

**19.** The method according to any one of claims 16 to 18 characterized in that said second excitation signal and said second emitted signal are modulated, and said analyzing step includes determining at least one of

- the extent of the phase shift and
- the magnitude of the relative demodulation between said modulated second excitation signal and said modulated second emitted signal, at least one of said extent and said magnitude being dependent on the contraction of said analyte in said medium.

**20.** The method according to any one of claims 16 to 19 characterized in that said sensing element is caused to provide said first emitted signal and said analyzing step includes analyzing both said first emitted signal and said second emitted signal in determining the concentration of said analyte in said medium.

**21.** The method according to any one of claims 16 to 20 characterized in that said analyzing step includes determining the ratio of said second emitted signal to said first emitted signal, said ratio being dependent on the concentration of said analyte in said medium.

**22.** The method according to any one of claims 16 to 21 characterized in that said first emitted signal and said second emitted signal are modulated and said analyzing step includes determining at least one of

- the extent of the phase shift between and
- the ratio of demodulation factors of said modulated first emitted signal and said modulated second emitted signal, at least one of said extent and said ratio being dependent on the concentration of said analyte in said medium.

**23.** The method according to any one of claims 16 to 22 characterized in that said medium is blood.

**24.** The method according to any one of claims 16 to 23 characterized in that said monomeric indicator components in enforced association are selected from the group consisting of intramolecular exciplex component forming molecules dispersed in a matrix material, intramolecular exciplex component forming molecules covalently attached, adsorbed or otherwise attached to the surface of a matrix material, intramolecular exciplex component forming molecules covalently attached to a matrix material, monomeric component aggregates covalently attached to a matrix material, monomeric component aggregates adsorbed to the surface of a matrix material, an inclusion complex comprising a supra-molecule, and ionically bound monomeric components.

**25.** A composition useful for measuring the concentration of an analyte in a medium comprising:

- a solid matrix material (20;120) which is permeable to said analyte in said medium, and
- two or more different monomeric components in enforced association at least one of which is a monomeric indicator component capable of providing a first emitted signal in response to being exposed to a first excitation signal,
- wherein said two or more different monomeric components are positioned or oriented in physical and/or molecular enforcement to promote or facilitate the formation and/or maintenance of a channel or path for exciplex component formation which (a) is kinetically dominant relative to free diffusion, and/or (b) is kinetically competitive with the decay of the exciplex component in the absence of the analyte,
- said composition being capable of providing a second emitted signal in response to being exposed to a second excitation signal, said second emitted signal being provided by an exciplex component produced from said monomeric components, said second emitted signal being dependent on the concentration of said analyte in said medium,
- wherein an exciplex component refers to an excited state species derived from a molecular or sub-molecular interaction of two or more different monomeric components at least one of which is a monomeric indicator component, one or more of which is a donor component and one or more of which is an acceptor component; and which satisfies the following relationship:

$$E_D^{ox} - E_A^{red} \leq h\nu(hex) + 0.25eV$$

where $E_D^{ox}$ is the ground state oxidation potential of the donor component, $E_A^{red}$ is the ground state reduction potential of the acceptor component, each measured relative to the same standard, hν (hex) is the maximum energy in eV (electron volts) of the excited state species measured in n-hexane, and the donor component and acceptor component are assigned so that the absolute value of $E_D^{ox} - E_A^{red}$ is minimized.

## Patentansprüche

**1.** Sensor zum Messen der Konzentration eines Analyten in einem Medium, mit:

- einem Sensorelement (20; 120) mit einem Matrixmaterial, das für den Analyten in dem Medium durchlässig ist, und zwei oder mehr verschiedenen Monomerkomponenten in Zwangsassoziation, von denen wenigstens eine eine Monomerindikatorkomponente ist, die geeignet ist, ein erstes Emissionssignal als Reaktion darauf, daß sie einem ersten Anregungssignal ausgesetzt ist, zu erzeugen,

- bei welchem die zwei oder mehr verschiedenen Monomerkomponenten physikalisch und/oder molekular derart zwangspositioniert oder ausgerichtet sind, daß sie die Bildung und/oder Erhaltung eines Kanals oder Weges zur Exciplex-Bildung fördern oder erleichtern, der (a) im Vergleich zu der freien Verteilung kinetisch dominant ist und/oder (b) mit dem Verfall der Exciplex-Komponente in Abwesenheit des Analyten kinetisch kompetitiv ist,

- wobei das das Sensorelement geeignet ist, ein zweites Emissionssignal als Reaktion darauf, daß es einem zweiten Anregungssignal ausgesetzt ist, zu liefern, wobei das zweite Emissionssignal von einer aus den Monomerkomponenten erzeugten Exciplex-Komponente geliefert wird,

- bei welchem eine Exciplex-Komponente auf eine Anregungszustandsart verweist, die durch eine molekulare oder submolekulare Interaktion von zwei oder mehr verschiedenen Monomerkomponenten hervorgerufen wird, von denen wenigstens eine eine Monomerindikatorkomponente ist, von denen eine oder mehr eine Donatorkomponente ist und von denen eine oder mehr eine Akzeptorkomponente ist; und die folgende Beziehung erfüllt:

$$E_D^{ox} - E_A^{red} \leq h\nu(hex) + 0{,}25\,eV$$

wobei $E_D^{ox}$ das Grundzustandsoxidationspotential der Donatorkomponente ist, $E_A^{red}$ das Grundzustandsreduktionspotential der Akzeptorkomponente ist, die jeweils im Vergleich zu demselben Standard gemessen sind, *hν(hex)* die Maximalenergie in eV (Elektronenvolt) der in n-Hexan gemessenen Anregungszustandsart ist, und die Donatorkomponente und die Akzeptorkomponente derart zugeordnet sind, daß der absolute Wert von $E_D^{ox} - E_A^{red}$ minimiert wird,

- einer Anregungsanordnung (14; 114), die derart angeordnet und geeignet ist, daß sie das zweite Anregungssignal an das Sensorelement liefert;

- einer Detektoranordnung (16; 116), die derart angeordnet und geeignet ist, daß sie das zweite Emissionssignal detektiert; und

- einer Prozessoranordnung (17; 117), die derart angeordnet und geeignet ist, daß sie das zweite Emissionssignal zum Bestimmen der Konzentration des Analyten in dem Medium analysiert.

**2.** Sensor nach Anspruch 1, dadurch gekennzeichnet, daß das zweite Emissionssignal von der Konzentration des Analyten in dem Medium abhängig ist.

**3.** Sensor nach Anspruch 1 oder 2, dadurch gekennzeichnet, daß das zweite Emissionssignal durch den Analyten in dem Medium dynamisch löschbar ist.

**4.** Sensor nach einem der Ansprüche 1 bis 3, dadurch gekennzeichnet. daß das zweite Emissionssignal von der Konzentration des Analyten in dem Medium in einem größeren Ausmaß als das erste Emissionssignal abhängig ist und das zweite Emissionssignal eine längere Wellenlänge als das erste Emissionssignal aufweist.

**5.** Sensor nach cinem der Ansprüche 1 bis 4, dadurch gekennzeichnet, daß das Matrixmaterial fest ist und die Monomerkomponenten an dem festen Matrixmaterial kovalent gebunden sind.

**6.** Sensor nach einem der Ansprüche 1 bis 5. dadurch gekennzeichnet. daß das erste Anregungssignal und das zweite Anregungssignal dasselbe Signal sind.

**7.** Sensor nach einem der Ansprüche 1 bis 5, dadurch gekennzeichnet, daß das zweite Anregungssignal und das zweite Emissionssignal moduliert sind und die Prozessoranordnung (17; 117) geeignet ist, wenigstens eines der folgenden Merkmale zu bestimmen:

- das Ausmaß der Phasenverschiebung und

- die Größenordnung der relativen Demodulation zwischen dem modulierten Anregungssignal und dem modulierten zweiten Emissionssignal, wobei wenigstens ein Merkmal aus der Gruppe des Ausmaßes und der Größenordnung von der Konzentration des Analyten in dem Medium abhängig ist.

**8.** Sensor nach einem der Ansprüche 1 bis 7, dadurch gekennzeichnet, daß die Anregungsanordnung (14; 114) derart angeordnet und geeignet ist, daß sie das erste Anregungssignal an das Sensorelement (20; 120) liefert; die Detektoranordnung (16; 116) derart angeordnet und geeignet ist, daß sie das erste Emissionssignal detektiert; und die Prozessoranordnung (17; 117) derart angeordnet ist, daß sie bei der Bestimmung der Konzentration des Analyten in dem Medium das erste Emissionssignal und das zweite Emissionssignal analysiert.

**9.** Sensor nach einem der Ansprüche 1 bis 8, dadurch gekennzeichnet, daß die Prozessoranordnung (17; 117) geeignet ist, das Verhältnis des zweiten Emissionssignals zu dem ersten Emissionssignal zu bestimmen, wobei das Verhältnis von der Konzentration des Analyten in dem Medium abhängig ist.

**10.** Sensor nach einem der Ansprüche 1 bis 8, dadurch gekennzeichnet, daß das erste Emissionssignal und das zweite Emissionssignal moduliert sind und die Prozessoranordnung (17; 117) geeignet ist, wenigstens eines der folgenden Merkmale zu bestimmen:

- das Ausmaß der Phasenverschiebung zwischen dem modulierten ersten Emissionssignal und dem modulierten zweiten Emissionssignal oder

- das Verhältnis der Demodulationsfaktoren des ersten Emissionssignals und des modulierten zweiten Emissionssignals,

wobei wenigstens ein Merkmal aus der Gruppe des Ausmaßes und des Verhältnisses von der Konzentration des Analyten in dem Medium abhängig ist.

**11.** Sensor nach einem der Ansprüche 1 bis 10, dadurch gekennzeichnet, daß der Analyt Sauerstoff ist.

**12.** Sensor nach einem der Ansprüche 1 bis 11, dadurch gekennzeichnet, daß die Monomerindikatorkomponenten in Zwangsassoziation aus der Gruppe ausgewählt sind, die aus in einem Matrixmaterial fein verteilten, eine intramolekulare Exciplexkomponente bildenden Molekülen, kovalent gebundenen, adsorbierten oder auf andere Weise an der Oberfläche eines Matrixmaterials angelagerten, eine intramolekulare Exciplexkomponente bildenden Molekülen, kovalent an einem Matrixmaterial gebundenen, eine intramolekulare Exciplexkomponente bildenden Molekülen, kovalent an einem Matrixmaterial gebundenen Monomerkomponentaggregaten, an der Oberfläche eines Matrixmaterials adsorbierten Monomerkomponentaggregaten, einer Einschlußverbindung mit einem Supramolekül und ionisch gebundenen Monomerkomponenten besteht.

**13.** Sensor nach einem der Ansprüche 1 bis 12, dadurch gekennzeichnet, daß die Monomerkomponenten durch weniger als 20 Atome verbunden sind.

**14.** Sensor nach einem der Ansprüche 1 bis 13, dadurch gekennzeichnet, daß die Monomerkomponenten durch zwischen 2 und 7 Kohlenstoffatome verbunden sind.

**15.** Sensor nach einem der Ansprüche 1 bis 14, dadurch gekennzeichnet, daß das zweite Emissionssignal von dem ersten Emissionssignal verschieden und unterschiedlich ist.

**16.** Verfahren zum Erfassen der Konzentration eines Analyten in einem Medium. mit den Schritten:

- Aussetzen eines Sensorelements (20; 120) dem Medium, wobei das Sensorelement (20; 120) ein Matrixmaterial, das für den Analyten in dem Medium durchlässig ist, und zwei oder mehr verschiedene Monomerkomponenten in Zwangsassoziation aufweist, von denen wenigstens eine eine Monomerindikatorkomponente ist. die geeignet ist, ein erstes Emissionssignal als Reaktion darauf, daß sie einem ersten Anregungssignal ausgesetzt ist, zu erzeugen,

- bei welchem die zwei oder mehr verschiedenen Monomerkomponenten physikalisch und/oder molekular derart zwangspositioniert oder ausgerichtet sind, daß sie die Bildung und/oder Erhaltung eines Kanals oder Weges zur Exciplex-Bildung fördern oder erleichtern, der (a) im Vergleich zu der freien Verteilung kinetisch dominant ist und/oder (b) mit dem Verfall der Exciplex-Komponente in Abwesenheit des Analyten kinetisch kompetitiv ist,

- wobei das Sensorelement (20; 120) geeignet ist, ein zweites Emissionssignal als Reaktion darauf, daß es einem zweiten Anregungssignal ausgesetzt ist, zu liefern, wobei das zweite Emissionssignal von einer aus den Monomerkomponenten erzeugten Exciplex-Komponente geliefert wird,

- bei welchem eine Exciplex-Komponente auf eine Anregungszustandsart verweist, die durch eine molekulare oder submolekulare Interaktion von zwei oder mehr verschiedenen Monomerkomponenten hervorgerufen wird, von denen wenigstens eine eine Monomerindikatorkomponente ist, von denen eine oder mehr eine Donatorkomponente ist und von denen eine oder mehr eine Akzeptorkomponente ist; und die folgende Beziehung erfüllt:

$$E_D^{ox} - E_A^{red} \leq h\nu(hex) + 0{,}25\,eV$$

wobei $E_D^{ox}$ das Grundzustandsoxidationspotential der Donatorkomponente ist, $E_A^{red}$ das Grundzustandsreduktionspotential der Akzeptorkomponente ist, die jeweils im Vergleich zu demselben Standard gemessen sind,

$h\nu$ (*hex*) die Maximalenergie in eV (Elektronenvolt) der in n-Hexan gemessenen Anregungszustandsart ist, und die Donatorkomponente und die Akzeptorkomponente derart zugeordnet sind. daß der absolute Wert von $E_D^{ox}$ - $E_A^{red}$ minimiert wird,

- wobei verursacht wird, daß das Sensorelement (20; 120) das zweite Emissionssignal liefert; und
- Analysieren des zweiten Emissionssignals zur Bestimmung der Konzentration des Analyten in dem Medium.

**17.** Verfahren nach Anspruch 16, dadurch gekennzeichnet, daß das zweite Emissionssignal von der Konzentration des Analyten in dem Medium in einem größeren Ausmaß als das erste Emissionssignal abhängig ist und das zweite Emissionssignal eine längere Wellenlänge als das erste Emissionssignal aufweist.

**18.** Verfahren nach Anspruch 16, dadurch gekennzeichnet, daß das Matrixmaterial fest ist und die Monomerkomponenten an dem festen Matrixmaterial kovalent gebunden sind.

**19.** Verfahren nach einem der Ansprüche 16 bis 18, dadurch gekennzeichnet, daß das zweite Anregungssignal und das zweite Emissionssignal moduliert sind und der Schritt des Analysierens es umfaßt, wenigstens eines der folgenden Merkmale zu bestimmen:

- das Ausmaß der Phasenverschiebung und
- die Größenordnung der relativen Demodulation zwischen dem modulierten Anregungssignal und dem modulierten zweiten Emissionssignal, wobei wenigstens ein Merkmal aus der Gruppe des Ausmaßes und der Größenordnung von der Konzentration des Analyten in dem Medium abhängig ist.

**20.** Verfahren nach einem der Ansprüche 16 bis 19, dadurch gekennzeichnet, daß verursacht wird, daß das Sensorelement das erste Emissionssignal liefert und der Schritt des Analysierens es umfaßt, bei der Bestimmung der Konzentration des Analyten in dem Medium sowohl das erste Emissionssignal als auch das zweite Emissionssignal zu analysieren.

**21.** Verfahren nach einem der Ansprüche 16 bis 20, dadurch gekennzeichnet, daß der Schritt des Analysierens es umfaßt, das Verhältnis des zweiten Emissionssignals zu dem ersten Emissionssignal zu bestimmen, wobei das Verhältnis von der Konzentration des Analyten in dem Medium abhängig ist.

**22.** Verfahren nach einem der Ansprüche 16 bis 21, dadurch gekennzeichnet, daß das erste Emissionssignal und das zweite Emissionssignal moduliert sind und der Schritt des Analysierens es umfaßt, wenigstens eines der folgenden Merkmale zu bestimmen:

- das Ausmaß der Phasenverschiebung zwischen dem modulierten ersten Emissionssignal und dem modulierten zweiten Emissionssignal oder
- das Verhältnis der Demodulationsfaktoren des ersten Emissionssignals und des modulierten zweiten Emissionssignals,

wobei wenigstens ein Merkmal aus der Gruppe des Ausmaßes und des Verhältnisses von der Konzentration des Analyten in dem Medium abhängig ist.

**23.** Verfahren nach einem der Ansprüche 16 bis 22, dadurch gekennzeichnet, daß das Medium Blut ist.

**24.** Verfahren nach einem der Ansprüche 16 bis 23, dadurch gekennzeichnet, daß die Monomerindikatorkomponenten in Zwangsassoziation aus der Gruppe ausgewählt sind, die aus in einem Matrixmaterial fein verteilten, eine intramolekulare Exciplexkomponente bildenden Molekülen, kovalent gebundenen, adsorbierten oder auf andere Weise an der Oberfläche eines Matrixmaterials angelagerten, eine intramolekulare Exciplexkomponente bildenden Molekülen, kovalent an einem Matrixmaterial gebundenen, eine intramolekulare Exciplexkomponente bildenden Molekülen, kovalent an einem Matrixmaterial gebundenen Monomerkomponentaggregaten, an der Oberfläche eines Matrixmaterials adsorbierten Monomerkomponentaggregaten, einer Einschlußverbindung mit einem Supramolekül und ionisch gebundenen Monomerkomponenten besteht.

**25.** Zuammensetzung, die zum Messen der Konzentration eines Analyten in einem Medium nützlich ist, mit:

- einem festen Matrixmaterial (20; 120), das für den Analyten in dem Medium durchläsig ist, und
- zwei oder mehr verschiedene Monomerkomponenten in Zwangsassoziation, von denen wenigstens eine eine Monomerindikatorkomponente ist. die geeignet ist, ein erstes Emissionssignal als Reaktion darauf, daß sie einem ersten Anregungssignal ausgesetzt ist, zu erzeugen,
- bei welchem die zwei oder mehr verschiedenen Monomerkomponenten physikalisch und/oder molekular derart zwangspositioniert oder ausgerichtet sind, daß sie die Bildung und/oder Erhaltung eines Kanals oder Weges zur Exciplex-Bildung fördern oder erleichtern, der (a) im Vergleich zu der freien Verteilung kinetisch dominant ist und/oder (b) mit dem Verfall der Exciplex-Komponente in Abwesenheit des Analyten kinetisch kompetitiv ist,
- wobei die Zusammensetzung geeignet ist, ein zweites Emissionssignal als Reaktion darauf, daß sie einem zweiten Anregungssignal ausgesetzt ist, zu liefern, wobei das zweite Emissionssignal von einer aus den Monomerkomponenten erzeugten Exciplex-Komponente geliefert wird, wobei das zweite Emissionssignal von der Konzentration des Analyten in dem Medium abhängt,
- bei welchem eine Exciplex-Komponente auf eine Anregungszustandsart verweist, die durch eine molekulare oder submolekulare Interaktion von zwei oder mehr verschiedenen Monomerkomponenten hervorgerufen wird, von denen wenigstens eine eine Monomerindikatorkomponente ist, von denen eine oder mehr eine Donatorkomponente ist und von denen eine oder mehr eine Akzeptorkomponente ist; und die folgende Beziehung erfüllt:

$$E_D^{ox} - E_A^{red} \leq h\nu\ (hex) + 0{,}25\,eV$$

- wobei $E_D^{ox}$ das Grundzustandsoxidationspotential der Donatorkomponente ist. $E_A^{red}$ das Grundzustandsreduktionspotential der Akzeptorkomponente ist, die jeweils im Vergleich zu demselben Standard gemessen sind, $h\nu$ (*hex*) die Maximalenergie in eV (Elektronenvolt) der in n-Hexan gemessenen Anregungszustandsart ist, und die Donatorkomponente und die Akzeptorkomponente derart zugeordnet sind, daß der absolute Wert von $E_D^{ox}$ - $E_A^{red}$ minimiert wird.

**Revendications**

**1.** Capteur de mesure de la concentration d'un analyte dans un milieu, comprenant :

- un élément sensible (20;120) comprenant un matériau de matrice perméable audit analyte dans ledit milieu, et deux ou plusieurs composants monomères différents en association forcée dont l'un au moins est un composant monomère indicateur capable de fournir un premier signal émis en réponse lorsqu'il est exposé à un premier signal d'excitation,
- dans lequel lesdits deux ou plusieurs composants monomères différents sont positionnés ou orientés de manière forcée au plan physique et/ou moléculaire afin de favoriser ou faciliter la formation et/ou le maintien d'un canal ou trajet de formation d'un composant exciplex qui (a) est prédominant du point de vue cinétique par rapport à la diffusion libre, et/ou (b) qui est en concurrence du point de vue cinétique avec la dégradation du composant exciplex en l'absence d'analyte,
- ledit élément sensible étant capable de fournir un deuxième signal émis en réponse lorsqu'il est exposé à un deuxième signal d'excitation, ledit deuxième signal émis étant fourni par un composant exciplex produit à partir desdits composants monomères,
- dans lequel un composant exciplex se réfère à une espèce à l'état excité provenant d'une interaction moléculaire ou submoléculaire de deux ou plusieurs composants monomères différents dont l'un au moins est un composant monomère indicateur, un ou plusieurs sont des composants donneurs, et un ou plusieurs sont des composants accepteurs ; et qui satisfait la relation suivante :

$$E_D^{ox} - E_A^{red} \leq h\nu(hex) + 0{,}25\ eV$$

dans laquelle $E_D^{ox}$ est le potentiel d'oxydation du composant donneur à l'état fondamental, $E_A^{red}$ est le potentiel de réduction du composant accepteur à l'état fondamental, mesurés chacun par rapport au même étalon, hν (hex) est l'énergie maximale en eV (électronvolts) de l'espèce à l'état excité mesurée dans le n-hexane, et le composant donneur ainsi que le composant accepteur sont spécifiés de manière à minimiser la valeur absolue de $E_D^{ox} - E_A^{red}$ ;

- un ensemble d'excitation (14;114) positionné et adapté pour fournir ledit deuxième signal d'excitation audit élément sensible ;
- un ensemble détecteur (16;116) positionné et adapté pour détecter ledit deuxième signal émis ; et
- un ensemble processeur (17;117) positionné et adapté pour analyser ledit deuxième signal émis afin de déterminer la concentration dudit analyte dans ledit milieu.

**2.** Capteur selon la revendication 1, caractérisé en ce que ledit deuxième signal émis dépend de la concentration dudit analyte dans ledit milieu.

**3.** Capteur selon la revendication 1 ou la revendication 2, caractérisé en ce que ledit deuxième signal émis peut être éteindu dynamiquement par ledit analyte dans ledit milieu.

**4.** Capteur selon l'une quelconque des revendications 1 à 3, caractérisé en ce que ledit deuxième signal émis dépend de la concentration dudit analyte dans ledit milieu dans une plus large mesure que ledit premier signal émis et en ce que la longueur d'onde dudit deuxième signal émis est plus grande que celle dudit premier signal émis.

**5.** Capteur selon l'une quelconque des revendications 1 à 4, caractérisé en ce que ledit matériau de matrice est solide et en ce que lesdits composants monomères sont liés de manière covalente audit matériau de matrice solide.

**6.** Capteur selon l'une quelconque des revendications 1 à 5, caractérisé en ce que ledit premier signal d'excitation et ledit deuxième signal d'excitation sont le même signal.

**7.** Capteur selon l'une quelconque des revendications 1 à 5, caractérisé en ce que ledit deuxième signal d'excitation et ledit deuxième signal émis sont modulés, et en ce que ledit ensemble processeur (17;117) est adapté pour déterminer au moins une des grandeurs suivantes :

- l'amplitude du déphasage, et
- la valeur de la démodulation relative entre ledit deuxième signal d'excitation modulé et ledit deuxième signal émis modulé, l'une au moins de ladite amplitude ou de ladite valeur étant dépendante de la concentration dudit analyte dans ledit milieu.

**8.** Capteur selon l'une quelconque des revendications 1 à 7, caractérisé en ce que ledit ensemble d'excitation (14; 114) est positionné et adapté pour fournir ledit premier signal d'excitation audit élément sensible (20;120) ; en ce que ledit ensemble détecteur (16;116) est positionné et adapté pour détecter ledit premier signal émis ; et en ce que ledit ensemble processeur (17;117) est positionné pour analyser ledit premier signal émis et ledit deuxième signal émis afin de déterminer la concentration dudit analyte dans ledit milieu.

**9.** Capteur selon l'une quelconque des revendications 1 à 8, caractérisé en ce que ledit ensemble processeur (17; 117) est adapté pour déterminer le rapport dudit deuxième signal émis sur ledit premier signal émis, ledit rapport dépendant de la concentration dudit analyte dans ledit milieu.

**10.** Capteur selon l'une quelconque des revendications 1 à 8, caractérisé en ce que ledit premier signal émis et ledit deuxième signal émis sont modulés et en ce que ledit ensemble processeur (17;117) est adapté pour déterminer au moins une des grandeurs suivantes :

- l'amplitude du déphasage entre, ou
- la valeur des coefficients de démodulation dudit premier signal émis modulé et dudit deuxième signal émis modulé, l'un au moins de ladite amplitude ou dudit rapport étant dépendant de la concentration dudit analyte dans ledit milieu.

**11.** Capteur selon l'une quelconque des revendications 1 à 10, caractérisé en ce que ledit analyte est l'oxygène.

**12.** Capteur selon l'une quelconque des revendications 1 à 11, caractérisé en ce que lesdits composants monomères

indicateurs en association forcée sont choisis dans le groupe comprenant des molécules formant un composant exciplex intramoléculaire dispersées dans un matériau de matrice, des molécules formant un composant exciplex intramoléculaire fixées Ce manière covalente, adsorbées ou fixées d'une autre façon sur la surface d'un matériau de matrice, des molécules formant un composant exciplex intramoléculaire fixées de manière covalente sur un matériau de matrice, des agrégats de composants monomères fixés de manière covalente à un matériau de matrice, des agrégats de composants monomères adsorbés sur la surface d'un matériau de matrice, un complexe d'inclusion comprenant une supramolécule et des composants monomères liés ioniquement.

**13.** Capteur selon l'une quelconque des revendications 1 à 12, caractérisé en ce que lesdits composants monomères sont liés par moins de 20 atomes.

**14.** Capteur selon l'une quelconque des revendications 1 à 13, caractérisé en ce que lesdits composants monomères sont liés par 2 à 7 atomes de carbone.

**15.** Capteur selon l'une quelconque des revendications 1 à 14, caractérisé en ce que le deuxième signal émis est différent et distinct du premier signal émis.

**16.** Procédé de détection de la concentration d'un analyte dans un milieu consistant à :

- exposer un élément sensible (20;120) audit milieu, ledit élément sensible (20;120) comprenant un matériau de matrice qui est perméable audit analyte dans ledit milieu, et deux ou plusieurs composants monomères différents en association forcée dont l'un au moins est un composant monomère indicateur capable de fournir un premier signal émis en réponse lorsqu'il est exposé à un premier signal d'excitation,
- dans lequel lesdits deux ou plusieurs composants monomères différents sont positionnés ou orientés de manière forcée au plan physique et/ou moléculaire afin de favoriser ou faciliter la formation et/ou le maintien d'un canal ou trajet de formation d'un composant exciplex qui (a) est prédominant du point de vue cinétique par rapport à la diffusion libre, et/ou (b) qui est en concurrence du point de vue cinétique avec la dégradation du composant exciplex en l'absence d'analyte,
- ledit élément sensible (20;120) étant capable de fournir un deuxième signal émis en réponse lorsqu'il est exposé à un deuxième signal d'excitation, ledit deuxième signal émis étant fourni par un composant exciplex produit à partir desdits composants monomères,
- dans lequel un composant exciplex se réfère à une espèce à l'état excité provenant d'une interaction moléculaire ou submoléculaire de deux ou plusieurs composants monomères différents dont l'un au moins est un composant monomère indicateur, un ou plusieurs sont des composants donneurs et un ou plusieurs sont des composants accepteurs ; et qui satisfait la relation suivante :

$$E_D^{ox} - E_A^{red} \leq h\nu(hex) + 0{,}25\ eV$$

  dans laquelle $E_D^{ox}$ est le potentiel d'oxydation du composant donneur à l'état fondamental, $E_A^{red}$ est le potentiel de réduction du composant accepteur à l'état fondamental, mesurés chacun par rapport au même étalon, hν (hex) est l'énergie maximale en eV (électronvolts) de l'espèce à l'état excité mesurée dans le n-hexane, et les composants donneur et accepteur sont spécifiés de manière à minimiser la valeur absolue de $E_D^{ox} - E_A^{red}$ ;
- faire en sorte que ledit élément sensible (20;120) fournisse ledit deuxième signal émis ; et
- analyser ledit deuxième signal émis afin de déterminer la concentration dudit analyte dans ledit milieu.

**17.** Procédé selon la revendication 16, caractérisé en ce que ledit deuxième signal émis dépend de la concentration dudit analyte dans ledit milieu dans une plus large mesure que ledit premier signal émis et en ce que la longueur d'onde dudit deuxième signal émis est plus grande que celle dudit premier signal émis.

**18.** Procédé selon la revendication 16 ou la revendication 17, caractérisé en ce que ledit matériau de matrice est solide et en ce que lesdits composants monomères sont liés de manière covalente audit matériau de matrice solide.

**19.** Procédé selon l'une quelconque des revendications 16 à 18, caractérisé en ce que ledit deuxième signal d'excitation et ledit deuxième signal émis sont modulés, et en ce que ladite étape d'analyse comprend la détermination d'au moins une des grandeurs suivantes :

- l'amplitude du déphasage, et

- la valeur de la démodulation relative entre ledit deuxième signal d'excitation modulé et ledit deuxième signal émis modulé, l'une au moins de ladite amplitude ou de ladite valeur étant dépendante de la concentration dudit analyte dans ledit milieu.

**20.** Procédé selon l'une quelconque des revendications 16 à 19, caractérisé en ce que l'on fait en sorte que ledit élément sensible fournisse ledit premier signal émis et en ce que ladite étape d'analyse comprend l'analyse à la fois dudit premier signal émis et dudit deuxième signal émis afin de déterminer la concentration dudit analyte dans ledit milieu.

**21.** Procédé selon l'une quelconque des revendications 16 à 20, caractérisé en ce que ladite étape d'analyse comprend la détermination du rapport dudit deuxième signal émis sur ledit premier signal émis, ledit rapport étant dépendant de la concentration dudit analyte dans ledit milieu.

**22.** Procédé selon l'une quelconque des revendications 16 à 21, caractérisé en ce que ledit premier signal émis et ledit deuxième signal émis sont modulés et en ce que ladite étape d'analyse comprend la détermination d'au moins une des grandeurs suivantes :

- l'amplitude du déphasage entre, ou
- la valeur des coefficients de démodulation dudit premier signal émis modulé et dudit deuxième signal émis modulé, l'un au moins de ladite amplitude ou du dit rapport étant dépendant de la concentration dudit analyte dans ledit milieu.

**23.** Procédé selon l'une quelconque des revendications 16 à 22, caractérisé en ce que ledit milieu est du sang.

**24.** Procédé selon l'une quelconque des revendications 16 à 23, caractérisé en ce que lesdits composants monomères indicateurs en association forcée sont choisis dans le groupe comprenant des molécules formant un composant exciplex intramoléculaire dispersées dans un matériau de matrice, des molécules formant un composant exciplex intramoléculaire fixées de manière covalente, adsorbées ou fixées d'une autre façon sur la surface d'un matériau de matrice, des molécules formant un composant exciplex intramoléculaire fixées de manière covalente sur un matériau de matrice, des agrégats de composants monomères fixés de manière covalente à un matériau de matrice, des agrégats de composants monomères adsorbés sur la surface d'un matériau de matrice, un complexe d'inclusion comprenant une supramolécule et des composants monomères liés ioniquement.

**25.** Composition utile pour mesurer la concentration d'un analyte dans un milieu, comprenant :

- un matériau de matrice (20;120) solide qui est perméable audit analyte dans ledit milieu, et
- deux ou plusieurs composants monomères différents en association forcée dont l'un au moins est un composant monomère indicateur capable de fournir un premier signal émis en réponse lorsqu'il est exposé à un premier signal d'excitation,
- dans laquelle lesdits deux ou plusieurs composants monomères différents sont positionnés ou orientés de manière forcée au plan physique et/ou moléculaire afin de favoriser ou faciliter la formation et/ou le maintien d'un canal ou trajet de formation d'un composant exciplex qui (a) est prédominant du point de vue cinétique par rapport à la diffusion libre, et/ou (b) qui est en concurrence du point de vue cinétique avec la dégradation du composant exciplex en l'absence d'analyte,
- ladite composition étant capable de fournir un deuxième signal émis en réponse lorsqu'elle est exposée à un deuxième signal d'excitation, ledit deuxième signal émis étant fourni par un composant exciplex produit à partir desdits composants monomères, ledit deuxième signal émis étant dépendant de la concentration dudit analyte dans ledit milieu,
- dans lequel un composant exciplex se réfère à une espèce à l'état excité provenant d'une interaction moléculaire ou submoléculaire de deux ou plusieurs composants monomères différents dont l'un au moins est un composant monomère indicateur, un ou plusieurs sont des composants donneurs et un ou plusieurs sont des composants accepteurs ; et qui satisfait la relation suivante :

$$E_D^{ox} - E_A^{red} \leq h\nu(hex) + 0,25 \text{ eV}$$

dans laquelle $E_D^{ox}$ est le potentiel d'oxydation du composant donneur à l'état fondamental, $E_A^{red}$ est le potentiel de réduction du composant accepteur à l'état fondamental, mesurés chacun par rapport au même étalon, hν(hex) est l'énergie maximale en eV (électronvolts) de l'espèce à l'état excité mesurée dans le n-hexane, et les composants donneur et accepteur sont spécifiés de manière à minimiser la valeur absolue de $E_D^{ox} - E_A^{red}$.

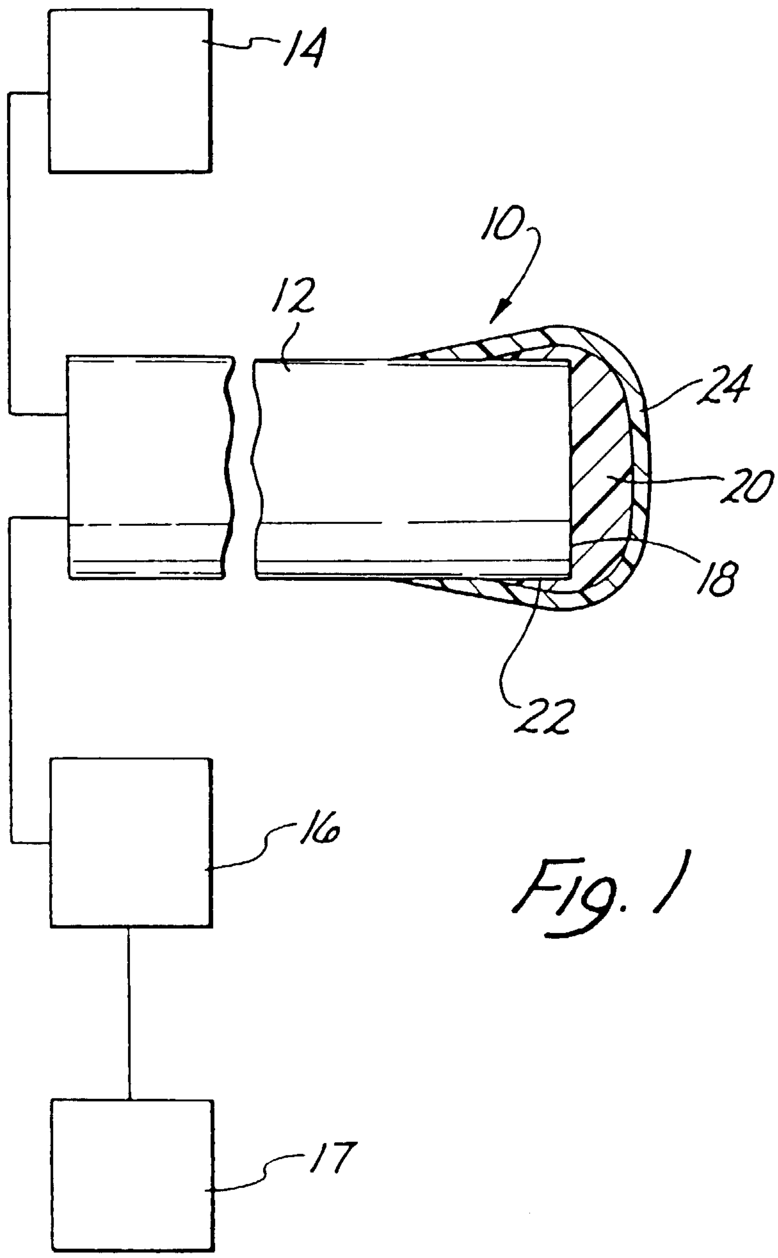


Fig. 1

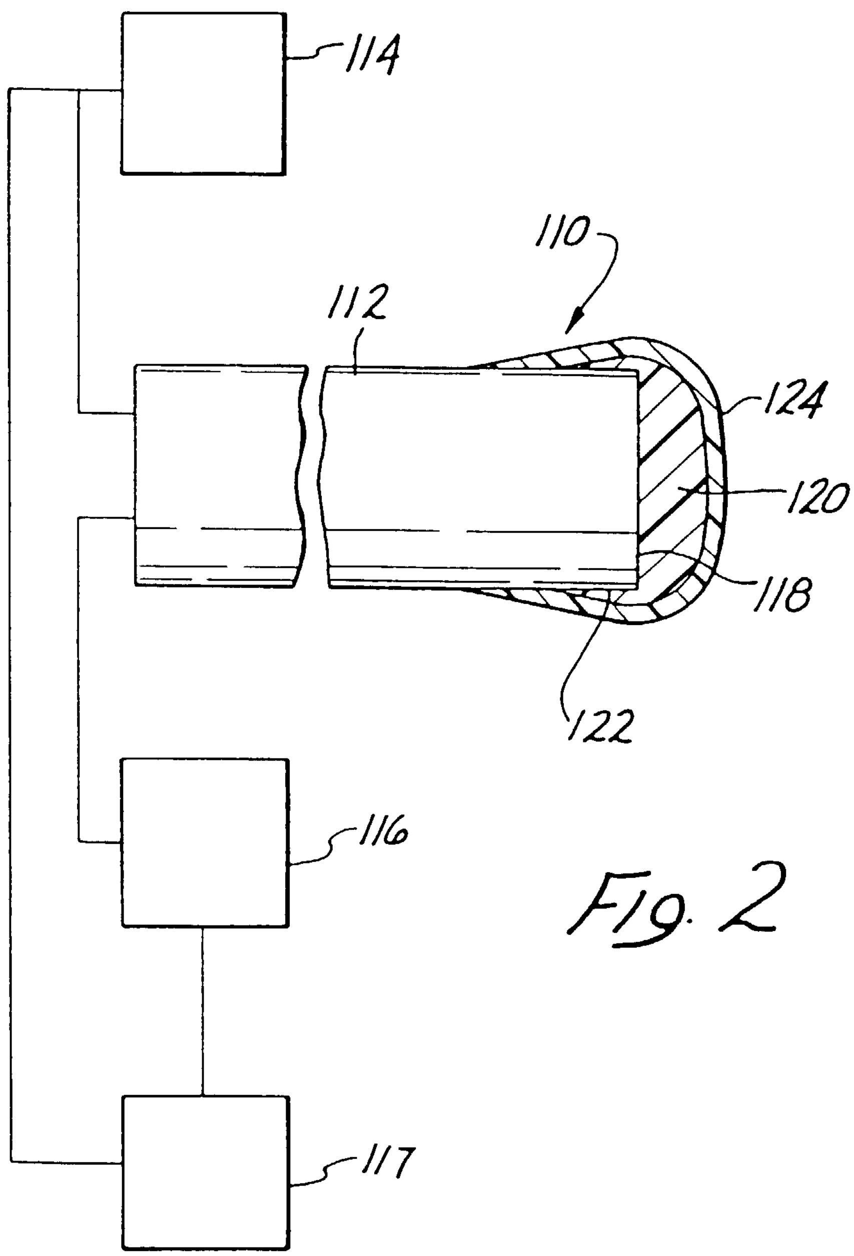


Fig. 2